(19) Europäisches Patentamt
European Patent Office
Office européen des brevets

(11) **EP 3 672 561 B1**

(12) **EUROPEAN PATENT SPECIFICATION**

(45) Date of publication and mention
of the grant of the patent:
**07.01.2026 Bulletin 2026/02**

(21) Application number: **18750027.7**

(22) Date of filing: **18.07.2018**

(51) International Patent Classification (IPC):
*A61K 8/22* (2006.01)  *A61K 8/34* (2006.01)
*A61K 8/81* (2006.01)  *A61Q 11/02* (2006.01)

(52) Cooperative Patent Classification (CPC):
**A61K 8/22; A61K 8/345; A61K 8/8147; A61Q 11/02**

(86) International application number:
**PCT/US2018/042697**

(87) International publication number:
**WO 2020/018089 (23.01.2020 Gazette 2020/04)**

(54) **ORAL CARE COMPOSITION**

ZAHPFLEGEZUSAMMENSETZUNGEN

COMPOSITION DE SOINS BUCCO-DENTAIRES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**01.07.2020 Bulletin 2020/27**

(73) Proprietor: **Colgate-Palmolive Company
New York, NY 10022 (US)**

(72) Inventors:
• **CHEN, Xiang
Somerset, New Jersey 08873 (US)**
• **CHOPRA, Suman
Monroe, New Jersey 08831 (US)**

(74) Representative: **Wibbelmann, Jobst
Wuesthoff & Wuesthoff
Patentanwälte und Rechtsanwalt PartG mbB
Schweigerstrasse 2
81541 München (DE)**

(56) References cited:
**EP-A1- 2 883 529      WO-A2-2006/071675
US-A- 5 985 249      US-A- 6 106 812
US-B1- 6 309 625**

• **DATABASE GNPD [online] MINTEL; 20 March
2018 (2018-03-20), ANONYMOUS: "Tooth
Whitening System", XP055539785, retrieved
from www.gnpd.com Database accession no.
5488241**

**Description**

**BACKGROUND**

Oral care products with teeth whitening attributes use a variety of active ingredients to remove stains or otherwise whiten teeth. For example, the most commonly used teeth whitening active ingredients are peroxides, such as hydrogen peroxide.

In certain applications, oral care products may be used in combination with a light source to enhance the teeth whitening effects of the teeth whitening active ingredients. For example, a teeth whitening gel may be used with a mouth tray having a light source. An oxidizing action of the teeth whitening active ingredients may be enhanced by the effects of the light illuminating the teeth and/or the teeth whitening active ingredients.

Depending on the method of application, oral care products with teeth whitening attributes may be provided in a variety of forms, such as toothpastes, rinses, gels, and strips. For example, some teeth whitening gels are designed to be used with a mouth tray. The mouth tray helps keep the teeth whitening active ingredients in close contact with the teeth during the teeth whitening process.

Such conventional gels are formulated primarily to stay in contact with the teeth so that their active ingredients can act on the teeth, without regard to the considerations related to use with a light source.

Accordingly, it would be useful to develop oral care compositions, such as teeth whitening gels, with improved characteristics for use with light sources that nonetheless maintain other desired characteristics, such as whitening efficiency, consistency, and stability. EP 2 883 529 A1 discloses a dental bleaching gel composition with high electric conductivity. WO 2006/071675 A1 discloses oral care (toothpaste) amalgam composition having first and second aqueous phases, wherein the first phase has a measured pH of from about 7 to about 9 and contains 2,4,4'-trichloro-2'-hydroxydiphenyl ether and a methylvinyl ether-maleic anhydride copolymer antibacterial-enhancing agent for the ether, wherein the second aqueous phase has a measured pH of from about 3.5 to about 6.5 and contains hydrogen peroxide and an antioxidant for the hydrogen peroxide. US 6,106,068 A discloses a dual component tooth whitening composition, which composition contains a peroxide whitening and a second ingredient incompatible with the peroxide compound, the second ingredient and the peroxide compound each being incorporated in separate dentifrice components which are physically separated until dispensed for use, the components retaining their original physical state when in contact, the first component being a composition containing a peroxide whitening compound in a vehicle thickened with a combination of a particulated water insoluble inorganic compound and an organic thickener other than an alkylene oxide polymer, and the second component containing the ingredient incompatible with the peroxide. US 5,985,249 A discloses sticky dental composition which include a sticky, glue-like matrix material for treating teeth using such compositions. US 6,309,625 A discloses compositions and methods that include potassium nitrate for whitening and/or reducing tooth sensitivity.

**BRIEF SUMMARY**

This summary is intended merely to introduce a simplified summary of some aspects of one or more embodiments of the present disclosure, which is defined in the claims. Further areas of applicability of the present invention will become apparent from the detailed description provided hereinafter. This summary is not an extensive overview, nor is it intended to identify key or critical elements of the present teachings, nor to delineate the scope of the disclosure, which is defined in the claims. Rather, its purpose is merely to present one or more concepts in simplified form as a prelude to the detailed description below.

The foregoing and/or other aspects and utilities embodied in the present disclosure may be achieved by providing an oil-based ingredient free oral care composition, including oral care composition, comprising: about 3 weight % of a structurant comprising at least one of Carbopol 974 NF, Carbopol 980 NF, and Carbopol 971P NF, based on a total weight of the oral care composition; from 30 weight % to 35 weight % of a humectant comprising glycerin, based on the total weight of the oral care composition; from 6 weight % to 9 weight % of a whitening agent comprising hydrogen peroxide, based on the total weight of the oral care composition; and at least 40 weight % of a carrier comprising water, based on the total weight of the oral care composition, and a pH regulating or neutralizing agent; wherein the oral care composition has a pH from about 4 to about 6 as defined in the claims.

In another embodiment, all the ingredients of the oral care composition are orally acceptable and water-soluble.

The oral care composition lacks oil-based or non-water-soluble ingredients as defined in the claims.

The structurant includes at least one of Carbopol 974 NF, Carbopol 980 NF, and Carbopol 971P NF as defined in the claims.

In another embodiment, the structurant consists essentially of Carbopol 974 NF.

The oral care composition includes about 3 weight % of the structurant; from about 30 weight % to about 35 weight % of the humectant; and from about 6 weight % to about 9 weight % of the whitening agent as further defined in the claims, wherein the oral care composition optionally has a pH from about 4.8 to about 5.8.

In another embodiment, the oral care composition has a transmission rate of at least 68 % for light in the wavelength of about 400 nm to about 420 nm, and the oral care composition has a transmission loss rate of 1 % or less at the surface interface due to refraction of light in the wavelength of about 400 nm to about 420 nm.

In another embodiment, the oral care composition further includes 0.01 weight % or less of a stabilizer, based on the total weight of the oral care composition; from about 0.5 weight % to about 2.0 weight % of a pH regulating agent, based on the total weight of the oral care composition; from about 0.1 weight % to about 2.0 weight % of a flavorant, based on the total weight of the oral care composition; and from about 0.1 weight % to about 5.0 weight % of a hypersensitivity agent, based on the total weight of the oral care composition, wherein the flavorant is water-soluble and is not oil-based.

In another embodiment, the oral care composition includes 0.01 weight % or less stabilizer; from about 0.5 weight % to about 2.0 weight % of the pH regulating agent; wherein the stabilizer includes EDTA, the pH regulating agent includes NaOH, and the hypersensitivity agent includes potassium nitrate.

In another embodiment, the oral care composition has a static yield of about 50 Pa or more.

In another embodiment, a hydrogen peroxide content of the oral care composition is greater than about 70% of an initial hydrogen peroxide content of the oral care composition, after 8 weeks of aging at 40 °C.

In another embodiment, a hydrogen peroxide content of the oral care composition is greater than about 70% of an initial hydrogen peroxide content of the oral care composition, after 13 weeks of aging at 40 °C.

The foregoing and/or other aspects and utilities embodied in the present disclosure may be achieved by providing an oral care composition as defined in the claims, for example consisting essentially of about 3 weight % of a structurant including a carboxypolymethylene polymer, based on a total weight of the oral care composition; from about 30 weight % to about 35 weight % of a humectant including glycerin, based on the total weight of the oral care composition; from about 6 weight % to about 9 weight % of a whitening agent including hydrogen peroxide, based on the total weight of the oral care composition; and at least 40 weight % of an aqueous carrier, based on the total weight of the oral care composition, wherein all the ingredients of the oral care composition are orally acceptable and water-soluble, wherein the oral care composition has a static yield of about 30 Pa or more, and wherein a hydrogen peroxide content of the oral care composition is greater than about 70% of an initial hydrogen peroxide content of the oral care composition, after 13 weeks of aging at 40 °C.

In another embodiment, the oral care composition further includes 0.01weight % or less of a stabilizer including EDTA; from about 0.5 weight % to about 2.0 weight % of a pH regulating agent including NaOH; from about 0.1 weight % to about 2.0 weight % of a water-soluble flavorant; and from about 0.1 weight % to about 5.0 weight % of a hypersensitivity agent including potassium nitrate.

In another embodiment, the oral care composition has a pH from about 4.8 to about 5.8, wherein the oral care composition has a transmission rate of at least 68 % for light in the wavelength of about 400 nm to about 420 nm, wherein the oral care composition has a refraction index of about 1.39 or less for light in the wavelength of about 400 nm to about 420 nm, and wherein the oral care composition has a transmission loss rate of 2 % or less at a surface interface due to refraction of light in the wavelength of about 400 nm to about 420 nm

In another embodiment, the structurant includes Carbopol 974 NF.

In another embodiment, the oral care composition is a teeth whitening gel configured for use with a mouth tray and a light source.

## DETAILED DESCRIPTION

Reference will now be made in detail to the various embodiments in the present disclosure within the scope of the claims. The embodiments are described below to provide a more complete understanding of the components, processes, compositions, and apparatuses disclosed herein. Any examples given are intended to be illustrative, and not restrictive. However, it will be apparent to one of ordinary skill in the art that the invention may be practiced within the scope of the claims without these specific details. In other instances, well-known methods, procedures, and components have not been described in detail so as not to unnecessarily obscure aspects of the embodiments.

Throughout the specification and claims, the following terms take the meanings explicitly associated herein, unless the context clearly dictates otherwise. Phrases such as "in an embodiment," "in certain embodiments," and "in some embodiments" as used herein do not necessarily refer to the same embodiment(s), though they may. Furthermore, the phrases "in another embodiment" and "in some other embodiments" as used herein do not necessarily refer to a different embodiment, although they may. As described below, various embodiments may be readily combined, without departing from the scope of the present disclosure which is defined in the claims.

As used herein, the term "or" is an inclusive operator, and is equivalent to the term "and/or," unless the context clearly dictates otherwise. The term "based on" is not exclusive and allows for being based on additional factors not described, unless the context clearly dictates otherwise. In the specification, the recitation of "at least one of A, B, and C," includes embodiments containing A, B, or C, multiple examples of A, B, or C, or combinations of A/B, A/C, B/C, A/B/B/B/B/C, A/B/C, etc. In addition, throughout the specification, the meaning of "a," "an," and "the" include plural references.

The meaning of "in" includes "in" and "on."

It will also be understood that, although the terms first, second, etc. may be used herein to describe various elements, these elements should not be limited by these terms. These terms are only used to distinguish one element from another. For example, a first object, component, or step could be termed a second object, component, or step, and, similarly, a second object, component, or step could be termed a first object, component, or step, without departing from the scope of the invention. The first object, component, or step, and the second object, component, or step, are both, objects, components, or steps, respectively, but they are not to be considered the same object, component, or step. It will be further understood that the terms "includes," "including," "comprises" and/or "comprising," when used in this specification, specify the presence of stated features, steps, operations, elements, and/or components, but do not preclude the presence or addition of one or more other features, steps, operations, elements, components, and/or groups thereof. Further, as used herein, the term "if" may be construed to mean "when" or "upon" or "in response to determining" or "in response to detecting," depending on the context.

All physical properties that are defined hereinafter are measured at 20° to 25° Celsius unless otherwise specified.

When referring to any numerical range of values herein, such ranges are understood to include each and every number and/or fraction between the stated range minimum and maximum, as well as the endpoints. For example, a range of 0.5-6% would expressly include all intermediate values of, for example, 0.6%, 0.7%, and 0.9%, all the way up to and including 5.95%, 5.97%, and 5.99%, among many others. The same applies to each other numerical property and/or elemental range set forth herein, unless the context clearly dictates otherwise.

Additionally, all numerical values are "about" or "approximately" the indicated value, and take into account experimental error and variations that would be expected by a person having ordinary skill in the art. It should be appreciated that all numerical values and ranges disclosed herein are approximate values and ranges, whether "about" is used in conjunction therewith.

Unless otherwise specified, all percentages and amounts expressed herein and elsewhere in the specification should be understood to refer to percentages by weight. The amounts given are based on the active weight of the material.

With regard to procedures, methods, techniques, and workflows that are in accordance with some embodiments, some operations in the procedures, methods, techniques, and workflows disclosed herein may be combined and/or the order of some operations may be changed.

The present inventors have unexpectedly and surprisingly discovered that exposing stained teeth to light having or being within a specific range of wavelengths can enhance or maximize the effects of a teeth whitening gel used with a corresponding light source. The present inventors have also unexpectedly and surprisingly developed an oral care composition having improved light transmission characteristics and having desirable whitening efficiency, consistency, and stability characteristics. The oral care composition (e.g., teeth whitening gel) is formulated to allow large amounts of light to pass through the composition and arrive at the surface of the teeth, where the stains are located, while maintaining or improving whitening efficiency, consistency, and stability characteristics. This is in contrast to conventional teeth whitening gels, which allow little (e.g. less than 50%) or almost no light to pass through them.

The oral care composition includes a carrier, a whitening agent, a structurant, and a humectant as further defined in the claims. The oral care composition may also include at least one of a stabilizer, a pH regulator, a flavorant, and a hypersensitivity agent.

In some embodiments, the oral care composition may include additional ingredients common to oral care compositions, such as thickeners, tartar control agents, surfactants, colorants, dyes, and pigments. In another embodiment, the oral care composition may only include additional ingredients that do not materially affect the basic and novel characteristics of the oral care composition. For example, the oral care composition may consist essentially of a carrier, a whitening agent, a structurant, and a humectant, and additional ingredients that do not materially affect at least one or more of the light transmission, whitening efficiency, consistency, and stability characteristics of the oral care composition.

All ingredients used in the oral care compositions described herein should be orally acceptable. "Orally acceptable" means an ingredient which is present in the composition as described in an amount and form which does not render the composition unsafe, unpalatable, or otherwise unsuitable for use in the oral cavity.

In addition, in some embodiments, the oral care composition does not include oil-based ingredients. For example, the oral care composition does not include oil-based flavorants or sweeteners.

The oral care composition includes one or more orally acceptable carrier as further defined in the claims. In various embodiments, the carrier allows a significant percentage of light (e.g., > 50%) to pass through it. For example, the oral care composition may use water as the carrier. In other embodiments, the carrier may also include one or more of sorbitol, glycerin, PEG, xylitol, and mixtures thereof. In certain embodiments, the oral care composition includes from about 40 weight % to about 90 weight % carrier, based on the total weight of the oral care composition. In certain embodiments, the oral care composition includes at least 40 weight %, at least 50 weight %, or at least 60 weight % carrier, based on the total weight of the oral care composition.

The carrier includes water as further defined in the claims, and all the other ingredients of the oral care composition are optionally water-soluble. In other embodiments, the oral care composition may include water-insoluble particles which are fine enough not to substantially scatter light transmission through the oral care composition. In certain embodiments, the oral care composition does not include non-water-soluble ingredients.

The oral care composition includes one or more whitening agent as further defined in the claims. As used herein, a "whitening agent" is a material that affects whitening of a tooth surface to which it is applied. For example, in some embodiments, the whitening agent is an oxidizing agent. In its broadest sense, "oxidizing agent" is intended to include those compounds which may accept an electron from another molecule in the environment of the oral cavity without having a deleterious or unacceptably harmful effect on the oral cavity in normal and accepted use. In various embodiments, the whitening agent allows a significant percentage of light (e.g., > 50%) to pass through it.

The whitening agent comprises hydrogen peroxide as further defined in the claims. In some embodiments, the whitening agent may include peroxides and hydroperoxides, such as hydrogen peroxide, peroxides of alkali and alkaline earth metals, peroxymonosulfate and peroxydisulfate salt, organic peroxy compounds, peroxy acids, salts thereof, and mixtures thereof. The whitening agent may include peroxides of alkali and alkaline earth metals include lithium peroxide, potassium peroxide, sodium peroxide, magnesium peroxide, calcium peroxide, barium peroxide, and mixtures thereof. The whitening agent may include organic peroxy compounds include urea peroxide, carbamide peroxide (also known as urea hydrogen peroxide), glyceryl hydrogen peroxide, alkyl hydrogen peroxides, dialkyl peroxides, alkyl peroxy acids, peroxy esters, diacyl peroxides, benzoyl peroxide, and monoperoxyphthalate, and mixtures thereof. The whitening agent may include peroxy acids and their salts include organic peroxy acids such as alkyl peroxy acids, and monoperoxyphthalate and mixtures thereof, as well as inorganic peroxy acid salts such as percarbonate, perphosphate, perborate and persilicate salts of alkali and alkaline earth metals such as lithium, potassium, sodium, magnesium, calcium and barium, and mixtures thereof.

The oral care composition includes from about 6 weight % to about 9 weight % whitening agent based on a total weight of the oral care composition as further defined in the claims.

The oral care composition includes one or more structurants as further defined in the claims. As used herein, the term structurant refers to a material that may not only thicken the oral care composition, but may also maintain the oral care composition in a homogenous state. That is, one where phase separation is minimized over time. In various embodiments, the structurant when properly incorporated into the carrier allows a significant percentage of light (e.g., > 50%) to pass through it.

In one embodiment, the structurant includes one or more polymers from the group of the crosslinked polyacrylic acids (carbopols), carrageenans (e.g., Irish moss, carrageenan, iota-carrageenan, *etc.*), high molecular weight polyethylene glycols *(e.g.,* CARBOWAX®, which is commercially available from The Dow Chemical Company of Midland, MI), cellulosic polymers, hydroxyethylcellulose, carboxymethylcellulose, and salts thereof *(e.g.,* CMC sodium), natural gums *(e.g.,* karaya, xanthan, gum arabic, and tragacanth), colloidal magnesium aluminum silicate, and the like, and mixtures or combinations thereof. The structurant includes at least one of Carbopol 974 NF, Carbopol 980 NF, and Carbopol 971P NF as defined in the claims, e.g. a carboxypolymethylene polymer and a suitable structurant may include Carbopol 974 NF, Carbopol 980 NF, and Carbopol 971P NF, available commercially from Lubrizol Corp., headquartered in Wickliffe, Ohio. In one preferred embodiment, the structurant includes Carbopol 974 NF.

The oral care composition includes about 3 weight % structurant as defined in the claims.

The oral care composition includes one or more humectants as further defined in the claims. The humectant may include glycerin, diols, and polyhydric alcohols. In one preferred embodiment, the humectant includes glycerin. The oral care composition includes from 10 weight % to 50 weight %, or from about 30 weight % to 35 weight % humectant, based on a total weight of the oral care composition. For example, the oral care composition may include from about 30 weight % to about 35 weight % glycerin.

The oral care composition may include one or more stabilizers. The stabilizer may help prevent premature activation or deterioration of the whitening agent and/or provide antibacterial effects. The one or more stabilizer may include edetic acid (EDTA) or salts of EDTA, polyphosphate salts, and butylated hydroxytoluene (BHT). In one preferred embodiment, the stabilizer includes EDTA.

The oral care composition may include 2.0 weight % or less stabilizer, based on a total weight of the oral care composition. For example, the oral care composition may include 0.02 weight % or less EDTA. In one embodiment, the oral care composition includes 0.01 weight % or less EDTA.

The oral care composition may include one or more pH regulating or neutralizing agents. For example, the pH regulating agent may include sodium hydroxide (NaOH). In other embodiments, the pH regulating agent may also include one or more of potassium hydroxide, ammonium hydroxide, arginine, amines (e.g. triethanolamine, diisopropanolamine, etc.). The oral care composition may include from about 0.5 weight % to about 2.0 weight % pH regulating agent, based on a total weight of the oral care composition. For example, the oral care composition may include from about 0.5 weight % to about 2.0 weight % NaOH.

The oral care composition may include one or more non-oil-based flavorants or sweeteners, such as sodium saccharin or aspartame. For example, the oral care composition may include from about 0.1 weight % to about 3.0 weight % sweetener, based on a total weight of the oral care composition. Sweeteners among those useful herein include orally acceptable natural or artificial, nutritive or non-nutritive sweeteners. However, as described above, the sweetener should be water-soluble and not oil-based.

The oral care composition may also include one or more hypersensitivity or desensitizing agents. In one preferred embodiments, the hypersensitivity agent includes potassium nitrate. For example, the oral care composition may include from about 0.1 weight % to about 5.0 weight %, from about 1 weight % to about 4 weight %, or from about 2 weight % to about 3 weight %, hypersensitivity agent, based on a total weight of the oral care composition.

The oral care composition may also include one or more anti-caries agents. The anti-caries agents may include a fluoride source, such as sodium fluoride. For example, the oral care composition may include from about 0.1 weight % to about 5.0 weight %, from about 1 weight % to about 4 weight %, or from about 2 weight % to about 3 weight %, anti-caries agent, based on a total weight of the oral care composition.

## EXAMPLES

Aspects of the present disclosure may be further understood by referring to the following examples. The examples are illustrative, and are not intended to be limiting embodiments thereof. Table 1 illustrates an oral care composition according to embodiments of the present disclosure.

Table 1

| Oral Care Composition Formula ||
| Ingredient | Weight % |
| Carbopol 974 NF | 2.0 % - 6.0 % |
| Glycerin | 30.0 % - 40.0 % |
| Hydrogen Peroxide | 0.1 % - 15.0 % |
| Potassium Nitrate | 0.1 % - 5.0 % |
| Water-soluble sweetener | 0.1 % - 2.0 % |
| Sodium Hydroxide | 0.87 % |
| EDTA | 0.01 % or less |
| Deionized Water | q.s. |

As described above, an oral care composition, such as a teeth whitening gel, is applied to the surface of teeth, and may be used in combination with a light source. Accordingly, in order to maximize the amount of light energy reaching the teeth (and any stains on the teeth), it is important for the oral care composition to have a high degree of light transmission (e.g., > 50% light transmission), especially of blue or actinic light. In particular, the inventors have discovered that light in the wavelength of about 400 nm to about 420 nm, which is within the blue light range, is especially beneficial for enhancing an oxidizing action of the whitening agents in the oral care composition.

Table 2 illustrates the light transmission of oral care compositions created according to Table 1. In particular, Oral Care Compositions #1 and #2 were created to have the same ingredients and amounts, except that the amount of hydrogen peroxide and potassium nitrate was varied as detailed below. Comparative Composition # 3 is similar to Oral Care Composition #1 except that Comparative Composition #3 included an oil-based flavorant. The light transmission was tested using a Beckman DU 520 UV-Visible light spectrophotometer. A further composition of Oral Care Composition #1 is also illustrated in Table 3 below.

Table 2

| Composition # | Light Transmission % ||||| 
| | 400 nm | 405 nm | 410 nm | 415 nm | 420 nm |
| Oral Care Composition #1 9.0% HP 3.0 % KNO3 | 68.08 | 68.56 | 69.13 | 69.70 | 70.11 |
| Oral Care Composition #2 7.5 % HP 0 % KNO3 | 58.55 | 58.58 | 58.63 | 58.66 | 58.66 |

(continued)

| Composition # | Light Transmission % | | | | |
|---|---|---|---|---|---|
| | 400 nm | 405 nm | 410 nm | 415 nm | 420 nm |
| Comparative Composition #3<br>9.0% HP<br>3.0 % KNO3<br>0.3% Oil-based flavorant | 32.95 | 33.09 | 33.23 | 33.35 | 33.43 |

As illustrated in Table 2, Oral Care Compositions #1 and #2 have a high transmission percentage for light in the wavelength of about 400 nm to about 420 nm. In particular, Oral Care Compositions #1 and #2 displayed at least 50 % transmission of 400-420 nm light. In some cases, Oral Care Compositions #1 and #2 displayed at least 60 % transmission, or even at least 70 % transmission, of 400-420 nm light. In contrast, as illustrated by Comparative Composition #3, the inclusion of an oil-based flavorant lowered the transmission percentage to less than 40 %.

Accordingly, in one embodiment, the oral care composition has a transmission rate of at least 50 % for light in the wavelength of about 400 nm to about 420 nm. In other embodiments, the oral care composition has a transmission rate of at least 60 % for light in the wavelength of about 400 nm to about 420 nm, or a transmission rate of at least 70 % for light in the wavelength of about 400 nm to about 420 nm.

For example, the oral care composition may have a transmission rate of at least 50 %, of at least 60%, or of at least 70% for light in the wavelength of about 405 nm to about 420 nm. The oral care composition may also have a transmission rate of at least 50 %, of at least 60%, or of at least 70% for light in the wavelength of about 410 nm to about 420 nm, or for light in the wavelength of about 415 nm to about 420. In one embodiment, the oral care composition has a transmission rate of at least 50 %, of at least 55 %, of at least 58 %, of at least 60%, of at least 65 %, of at least 68 %, or of at least 70% for light in the wavelength of about 420 nm.

In addition to transmission rate, light scattering (e.g., reflecting or bending) at surface interphases also affects the amount of light that may reach the teeth surface. For example, as light initially contacts the surface of the oral care composition from the light source, light scattering at the surface of the oral care composition diminishes the total amount of light available to travel through the oral care composition and ultimately to illuminate the teeth surface. The amount of light scattering at the interphase surface can be measured as the refractive index.

Table 3 illustrates a composition of Oral Care Composition #1 and Oral Care Compositions #4 and 5. Table 4 illustrates the refractive index and associated transmission loss for oral care compositions according to embodiments in the present disclosure. In particular, the refractive index and transmission loss of Oral Care Composition #1 was compared to Oral Care Compositions #4 and 5. While not intending to be bound by any particular theory, the inventors discovered that the carriers and humectants affected it the most, in the exemplary oral care composition the amount of glycerin and water may have the largest impact on the refraction index and associated transmission loss for the oral care compositions.

Oral Care Compositions #4 and 5 were created to have the same ingredients and amounts as Oral Care Composition #1, except that the amount of glycerin (and therefore also the total amount of water) was varied as detailed below. The refractive index and transmission loss was measured by a bench-top refractometer, assuming that the loss of light happened at a normal incidence condition and with the refractive index of the light source being 1.687.

The transmission loss was calculated as the reflectivity coefficients of two mediums under the following formula:

## Formula 1

$$R = [(n_2-n_1)/(n_2+n_1)]^2$$

wherein R is the transmission loss and n1 and n2 are light sourcing refractive index being 1.687 and composition index as listed in the Table 4, respectively.

Table 3

| Oral Care Composition Formulas | | | |
|---|---|---|---|
| | Oral Care Composition #1 | Oral Care Composition #4 | Oral Care Composition #5 |
| Ingredient | Weight % | Weight % | Weight % |
| Carbopol 974 NF | 3.0% | 3.0% | 3.0% |
| Glycerin | 30.0 % | 33.0% | 35.0% |

(continued)

| Oral Care Composition Formulas | | | |
|---|---|---|---|
| | Oral Care Composition #1 | Oral Care Composition #4 | Oral Care Composition #5 |
| Ingredient | Weight % | Weight % | Weight % |
| Hydrogen Peroxide | 9.0% | 9.0% | 9.0% |
| Potassium Nitrate | 3.0% | 3.0% | 3.0% |
| Water-soluble sweetener | 2.0 % | 2.0 % | 2.0 % |
| Sodium Hydroxide | 0.875% | 0.875% | 0.875% |
| Deionized Water | q.s. | q.s. | q.s. |

Table 4

| Composition # | Transmission loss due to light reflection | |
|---|---|---|
| | Refractive Index | Transmission Loss % |
| Oral Care Composition #1 30 % Glycerin | 1.3864 | 0.9566 |
| Oral Care Composition #4 33 % Glycerin | 1.3886 | 0.9413 |
| Oral Care Composition #5 35 % Glycerin | 1.3918 | 0.9193 |

As illustrated in Table 4, Oral Care Compositions #1, #4, and #5 have a low transmission loss due to refraction for light in the wavelength of about 400 nm to about 420 nm. In particular, Oral Care Compositions #1 and #4-5 displayed 2% or less transmission loss at the interface between the light source and the oral care composition for light in the wavelength of about 400 nm to about 420 nm. In addition, Oral Care Compositions #1 and #4-5 displayed a refractive index of about 1.39 or less for light in the wavelength of about 400 nm to about 420 nm.

Accordingly, in one embodiment, the oral care composition has a refraction index of about 1.39 or less for light in the wavelength of about 400 nm to about 420 nm. In another embodiment, the oral care composition has a refraction index of about 1.40 or less for light in the wavelength of about 400 nm to about 420 nm. In other embodiments, the oral care composition has a transmission loss rate of 2 % or less at the surface interface due to refraction for light in the wavelength of about 400 nm to about 420 nm, or a transmission loss rate of 1 % or less at the surface interface due to refraction for light in the wavelength of about 400 nm to about 420 nm.

Generally, viscosity or consistency is an important parameter for oral care compositions, such as teeth whitening gels. For example, it is important for the teeth whitening gel to stay in place against the teeth or within a tray during the teeth whitening application, and a composition having a high static yield stress will not as readily flow off of the teeth as compared to a composition with lower static yield stress.

Table 5 illustrates a static yield stress for oral care compositions according to embodiments in the present disclosure. In particular, Table 5 compares the static yield of Oral Care Compositions #1 and #2 to Comparative Composition #6.

Comparative Compositions #6 was created to have the same ingredients and amounts as Oral Care Composition #1, except that the amount of Carbopol 974 NF was reduced to 1.5 % as detailed below. The static yield was measured using a Brookfield viscometer (model HADV-II+Pro) equipped with fitflow software.

Table 5

| Composition # | Static Yield (Pa) |
|---|---|
| Oral Care Composition #1<br>9.0% HP<br>3.0 % KNO3<br>3.0 % Carbopol 974 NF | 52.7 |
| Oral Care Composition #2<br>7.5 % HP<br>0 % KNO3<br>3.0 % Carbopol 974 NF | 132 |

(continued)

| Composition # | Static Yield (Pa) |
|---|---|
| Comparative Composition #6<br>9.0 % HP<br>3.0 % KNO3<br>1.5 % Carbopol 974 NF | 10.7 |

As illustrated in Table 5, Oral Care Compositions #1 and 2 have a static yield above 30 Pa. This indicates a relatively high viscosity or thick consistency for the oral care composition, and was verified when Oral Care Compositions #1 and 2 moved very little inside of a tray even when submerged in water for up to 30 minutes. In contrast, Comparative Composition #6 had a static yield stress of 10.7 Pa indicating a very liquid or runny or low viscosity composition.

Accordingly, in one embodiment, the oral care composition has a static yield of about 30 Pa or more. In other embodiments, the oral care composition has a static yield of about 50 Pa or more.

In certain embodiments, a viscosity of the oral care composition is from about 50,000 centipoise (cPs) to about 500,000 cPs at 25° C. In other embodiments, the oral care composition has a viscosity of 100,000 cPs or more, 300,000 cPs or more, or 500,000 cPs or more at 25° C.

The potential effectiveness of oral care compositions, such as teeth whitening gels, can be measured in terms of active content, that is, active hydrogen peroxide content in the oral care composition. Accordingly, the stability of the oral care composition can be determined by monitoring the change of hydrogen peroxide level over time. The level of hydrogen peroxide can be obtained experimentally by a well-known thiosulfate based iodine titration method.

Table 6 illustrates the stability of oral care compositions according to embodiments in the present disclosure. In particular, Table 6 illustrates the stability of Oral Care Compositions #1 and #2 in terms of hydrogen peroxide content over time.

The amount of loss in hydrogen peroxide content in the oral care compositions of Table 6 were determined via this titration method as follows: about 1.3 grams of the Table 6 compositions were added to a beaker. 25 ml of glacial acetic acid was then added, followed by addition of 50 ml of ethanol/water (1:1 volume ratio "v/v"). The mixture was then mixed until the Table 6 compositions were fully suspended. 5 ml of 20% (by weight) potassium iodide solution and 4 drops of ammonium molybdate solution were added to the mixture and mixed for 5 minutes. The mixture turned yellowish. 2 ml of a starch indicator was added to the mixture. The mixture turned dark brown in color. The mixture was then titrated with 0.1 N sodium thiosulfate solution until the dark color disappeared and the amount (mL) of sodium thiosulfate solution used was recorded. The percentage of hydrogen peroxide was then calculated as follows:

Formula 2

$$(\%HP) = \frac{(ml\ thiosulfate\ used) \times (N\ of\ thiosulfate) \times (meq\ wt\ of\ oxidizing\ agent) \times (100)}{weight\ of\ sample\ (g)}$$

where the meq wt of the oxidizing agent is 0.01701 representing hydrogen peroxide.

To determine the stability of the oral care compositions, the %HP was measured before aging and after aging at 40 °C and 75 % relative humidity (RH) as illustrated in Table 6. The oral care compositions were aged up to 13 weeks, which is the extent of time commonly used to predict a 24 month shelf life under normal conditions. Under normal conditions, maintenance of about 70% or more of the initial %HP after 8 weeks is considered a good result, and signifies that the composition will be sufficiently stable for commercial distribution and sales, and maintenance of about 70% or more of the initial %HP after 13 weeks is considered a surprisingly good result.

Table 6

| | AO% | pH | Remaining HP % after 4 weeks | Remaining HP % after 8 weeks | Remaining HP % after 13 weeks |
|---|---|---|---|---|---|
| Oral Care Composition #1 | 5.2 | 8.4 % | 7.8 % | 7.6 % | |
| Oral Care Composition #2 | 5.6 | 7.4 % | 6.9 % | 6.8 % | |

As illustrated in Table 6, Oral Care Compositions #1 and #2 had less than a 30 % drop in %HP during the 13-week accelerated aging study. This result indicates a very good stability.

Accordingly, in some embodiments, hydrogen peroxide content of the oral care composition is greater than about 70%, about 80%, or about 90%, of an initial hydrogen peroxide content of the oral care composition after 8 weeks of aging at 40 °C. In another embodiment, a hydrogen peroxide content of the oral care composition is greater than about 70%, about 80%, or about 90%, of an initial hydrogen peroxide content of the oral care composition after 13 weeks of aging at 40 °C.

While not intending to be bound by any theory, the inventors discovered that controlling the pH of the oral care composition contributed to its stability. Accordingly, in some embodiments, the pH of the oral care composition is between about 4 and about 6. In a preferred embodiment, the pH of the oral care composition is between about 4.8 and about 5.8.

As described above, in some embodiments, the oral care compositions disclosed herein have an enhanced whitening efficiency when used together with a light source in the wavelength of about 400 nm to about 420 nm. As used herein, the phrase "whitening efficacy" is intended to refer to the amount of change in tooth color. The color change can be measured according to the L*a*b* color scale. The luminance or lightness (L*) value measures brightness and varies from a value of one hundred for perfect white to zero for black, assuming a* and b* are zero. The a* value is a measure of redness when positive, gray when zero and greenness when negative. The b* value is a measure of yellowness when positive, gray when zero and blueness when negative. Generally, teeth appear whiter as: the L* value increases meaning they become brighter, the a* value increases or decreases, depending upon whether the stained teeth have a green tint or red tint prior to whitening, and the b* value decreases meaning they become less yellow. While this is the general relationship for perceived whitening, the b* value might also slightly increase if the magnitude of the increase of the L* value is large enough. Similarly, the L* value might also decrease if the magnitude of the decrease of the b* value is large enough to overshadow the less significant change in L*.

In some embodiments, the whitening index (W*) is used to assess tooth whiteness. The whiteness index is based on the distance of a color value from a nominal white point, represented in CIELAB color space as L* = 100, a* = 0 and b* = 0, and defined according to the following formula 3:

### Formula 3

$$W^* = [(a^*)^2+(b^*)^2+(L^*-100)^2]^{1/2}.$$

Changes in W* may be used to assess the whitening efficacy of a composition before and after a treatment. The following Formula 4 may be used to calculate $\Delta W^*$:

### Formula 4

$$\Delta W^* = W^*(Treatment) - W^* (baseline).$$

The whitening efficacy of oral care compositions according to embodiments of this disclosure was tested as follows: bovine teeth were placed in a holding tray and measured for L*,a*,b* values. Oral Care Composition #1 was applied to the teeth and then the holding tray was put into a black box, where a printed LED lamp was fixed on the top of the box with an open slot to allow the LED lamp to shed light onto the teeth. The LED lamp emitted blue light with a wavelength near about 410 nm. The gap between the LED lamp and the teeth was maintained at about 5 mm. The treatment time was 10 min, after which the teeth were cleaned with water and the L*,a*,b* values were measured. The treatment was repeated for a total of 10 cycles. A control treatment was performed similarly as above, except that the LED light was not turned on. The Whiteness index, W*, and the $\Delta W^*$ (compared to baseline W*) was used to record the change of whiteness after each whitening treatment cycle. The whiter the tooth after treatment, the more negative the $\Delta W^*$ value is.

Table 7 illustrates a whitening efficiency of an oral care composition when enhanced with light in wavelengths of about 400 nm to about 420 nm. In particular, Table 7 illustrates the whitening efficiency of Oral Care Composition #1 when used in combination with light in wavelengths of about 400 nm to about 420 nm as compared to without the light source.

Table 7.

| Treatment # | Oral Care Composition #1 without light | Oral Care Composition #1 with 400 nm - 420 nm light |
|---|---|---|
| | $\Delta W^*$ | $\Delta W^*$ |
| 1st treatment | -2.37 | -6.06 |
| 2nd treatment | -3.41 | -9.12 |
| 3rd treatment | -3.92 | -10.74 |
| 4th treatment | -4.46 | -11.62 |

(continued)

| Treatment # | Oral Care Composition #1 without light | Oral Care Composition #1 with 400 nm - 420 nm light |
|---|---|---|
| | ΔW* | ΔW* |
| 5th treatment | -4.78 | -12.09 |
| 6th treatment | -5.23 | -12.20 |
| 7th treatment | -6.95 | -13.94 |
| 8th treatment | -7.42 | -14.21 |
| 9th treatment | -7.67 | -14.40 |
| 10th treatment | -7.86 | -14.54 |

As illustrated in Table 7, Oral Care Composition #1 had a lower ΔW* value used in combination with light in wavelengths of about 400 nm to about 420 nm. Accordingly, Table 7 demonstrates the superior whitening efficacy of oral care composition according to embodiments of the present disclosure using light in wavelengths of about 400 nm to about 420 nm.

The present disclosure has been described with reference to exemplary embodiments. Although a few embodiments have been shown and described, it will be appreciated by those skilled in the art that changes may be made within the scope of the claims.

**Claims**

1. An oil-based ingredient free oral care composition, comprising:

    about 3 weight % of a structurant comprising at least one of Carbopol 974 NF, Carbopol 980 NF, and Carbopol 971P NF, based on a total weight of the oral care composition;
    from 30 weight % to 35 weight % of a humectant comprising glycerin, based on the total weight of the oral care composition;
    from 6 weight % to 9 weight % of a whitening agent comprising hydrogen peroxide, based on the total weight of the oral care composition; and
    at least 40 weight % of a carrier comprising water, based on the total weight of the oral care composition, and a pH regulating or neutralizing agent;
    wherein the oral care composition has a pH from about 4 to about 6.

2. The oral care composition of claim 1, wherein the structurant consists essentially of Carbopol 974 NF.

3. The oral care composition of claim 1, wherein the oral care composition has a pH from 4.8 to 5.8.

4. The oral care composition of claim 1, wherein the oral care composition has a transmission rate of at least 68 % for light in the wavelength of 400 nm to 420 nm, and
   wherein the oral care composition has a transmission loss rate of 1 % or less at the surface interface due to refraction of light in the wavelength of 400 nm to 420 nm.

5. The oral care composition of claim 1, further comprising:

    0.01 weight % or less of a stabilizer, based on the total weight of the oral care composition;
    from 0.5 weight % to 2.0 weight % of a pH regulating agent, based on the total weight of the oral care composition;
    from 0.1 weight % to 2.0 weight % of a flavorant, based on the total weight of the oral care composition; and
    from 0.1 weight % to 5.0 weight % of a hypersensitivity agent, based on the total weight of the oral care composition,
    wherein the flavorant is water-soluble and is not oil-based.

6. The oral care composition of claim 5, comprising:

    0.01 weight % or less stabilizer;

from 0.5 weight % to 2.0 weight % of the pH regulating agent;
wherein the stabilizer comprises EDTA, the pH regulating agent comprises NaOH, and the hypersensitivity agent comprises potassium nitrate.

7. The oral care composition of claim 1, consisting essentially of:

about 3 weight % of a structurant comprising at least one of Carbopol 974 NF, Carbopol 980 NF, and Carbopol 971P NF, based on a total weight of the oral care composition;
from 30 weight % to 35 weight % of a humectant comprising glycerin, based on the total weight of the oral care composition;
from 6 weight % to 9 weight % of a whitening agent comprising hydrogen peroxide, based on the total weight of the oral care composition; and
at least 40 weight % of an aqueous carrier, based on the total weight of the oral care composition,
wherein all the ingredients of the oral care composition are orally acceptable and water-soluble.

8. The oral care composition of claim 7, further comprising

0.01 weight % or less of a stabilizer comprising EDTA;
from 0.5 weight % to 2.0 weight % of a pH regulating agent comprising NaOH;
from 0.1 weight % to 2.0 weight % of a water-soluble flavorant; and
from 0.1 weight % to 5.0 weight % of a hypersensitivity agent comprising potassium nitrate.

9. The oral care composition of claim 7, wherein the structurant comprises Carbopol 974 NF.

10. The oral care composition of claim 7, wherein the oral care composition is a teeth whitening gel configured for use with a mouth tray and a light source.

**Patentansprüche**

1. Eine ölbasierte, frei von Inhaltsstoffen Mundpflegezusammensetzung, umfassend:
etwa 3 Gew.-% eines Strukturmittels, das mindestens eines der folgenden umfasst: Carbopol 974 NF, Carbopol 980 NF und Carbopol 971P NF, bezogen auf das Gesamtgewicht der Mundpflegezusammensetzung;

30 bis 35 Gew.-% eines Feuchthaltemittels, das Glycerin umfasst, bezogen auf das Gesamtgewicht der Mundpflegezusammensetzung;
6 bis 9 Gew.-% eines Weißmachers, der Wasserstoffperoxid umfasst, bezogen auf das Gesamtgewicht der Mundpflegezusammensetzung; und
mindestens 40 Gew.-% eines Trägers, der Wasser umfasst, bezogen auf das Gesamtgewicht der Mundpflegezusammensetzung, und
ein pH-regulierendes oder neutralisierendes Mittel;
wobei die Mundpflegezusammensetzung einen pH-Wert von etwa 4 bis etwa 6 aufweist.

2. Mundpflegezusammensetzung nach Anspruch 1, wobei das Strukturmittel im Wesentlichen aus Carbopol 974 NF besteht.

3. Mundpflegezusammensetzung nach Anspruch 1, wobei die Mundpflegezusammensetzung einen pH-Wert von 4,8 bis 5,8 aufweist.

4. Mundpflegezusammensetzung nach Anspruch 1, wobei die Mundpflegezusammensetzung eine Transmissionsrate von mindestens 68 % für Licht in der Wellenlänge von 400 nm bis 420 nm aufweist, und
wobei die Mundpflegezusammensetzung eine Transmissionsverlustrate von 1 % oder weniger an der Oberflächengrenzfläche aufgrund der Brechung von Licht in der Wellenlänge von 400 nm bis 420 nm aufweist.

5. Mundpflegezusammensetzung nach Anspruch 1, die ferner umfasst:

0,01 Gew.-% oder weniger eines Stabilisators, bezogen auf das Gesamtgewicht der Mundpflegezusammensetzung;

0,5 Gew.-% bis 2,0 Gew.-% eines pH-Regulierungsmittels, bezogen auf das Gesamtgewicht der Mundpflegezusammensetzung;

0,1 Gew.-% bis 2,0 Gew.-% eines Aromastoffs, bezogen auf das Gesamtgewicht der Mundpflegezusammensetzung; und

0,1 Gew.-% bis 5,0 Gew.-% eines Überempfindlichkeitsmittels, bezogen auf das Gesamtgewicht der Mundpflegezusammensetzung,

wobei der Aromastoff wasserlöslich und nicht auf Ölbasis ist.

6. Mundpflegezusammensetzung nach Anspruch 5, umfassend:

0,01 Gew.-% oder weniger Stabilisator;

0,5 Gew.-% bis 2,0 Gew.-% eines pH-Regulierungsmittels;

wobei der Stabilisator EDTA umfasst, der pH-Regulator NaOH umfasst und das Überempfindlichkeitsmittel Kaliumnitrat umfasst.

7. Mundpflegezusammensetzung nach Anspruch 1, bestehend im Wesentlichen aus:
etwa 3 Gew.-% eines Strukturmittels, das mindestens eines von Carbopol 974 NF, Carbopol 980 NF und Carbopol 971P NF umfasst, bezogen auf das Gesamtgewicht der Mundpflegezusammensetzung;

30 Gew.-% bis 35 Gew.-% eines Feuchthaltemittels, das Glycerin umfasst, bezogen auf das Gesamtgewicht der Mundpflegezusammensetzung;

6 bis 9 Gew.-% eines Weißmachers, der Wasserstoffperoxid umfasst, bezogen auf das Gesamtgewicht der Mundpflegezusammensetzung; und

mindestens 40 Gew.-% eines wässrigen Trägers, bezogen auf das Gesamtgewicht der Mundpflegezusammensetzung,

wobei alle Inhaltsstoffe der Mundpflegezusammensetzung oral verträglich und wasserlöslich sind.

8. Mundpflegezusammensetzung nach Anspruch 7, die ferner umfasst

0,01 Gew.-% oder weniger eines Stabilisators, der EDTA umfasst;

0,5 Gew.-% bis 2,0 Gew.-% eines pH-Regulierungsmittels, das NaOH umfasst;

0,1 Gew.-% bis 2,0 Gew.-% eines wasserlöslichen Aromastoffs; und

0,1 Gew.-% bis 5,0 Gew.-% eines Überempfindlichkeitsmittels, das Kaliumnitrat umfasst.

9. Mundpflegezusammensetzung nach Anspruch 7, wobei der Strukturgeber Carbopol 974 NF umfasst.

10. Mundpflegezusammensetzung nach Anspruch 7, wobei die Mundpflegezusammensetzung ein Zahnaufhellungsgel ist, das zur Verwendung mit einer Mundschiene und einer Lichtquelle konfiguriert ist.

**Revendications**

1. Composition de soins bucco-dentaires sans ingrédients à base d'huile, comprenant :

environ 3 % en poids d'un agent structurant comprenant au moins un élément parmi le Carbopol 974 NF, le Carbopol 980 NF et le Carbopol 971P NF, par rapport au poids total de la composition de soins bucco-dentaires ;
de 30 % en poids à 35 % en poids d'un humectant comprenant de la glycérine, par rapport au poids total de la composition de soins bucco-dentaires ;
de 6 % en poids à 9 % en poids d'un agent de blanchiment comprenant du peroxyde d'hydrogène, par rapport au poids total de la composition de soins bucco-dentaires ; et
au moins 40 % en poids d'un support comprenant de l'eau, par rapport au poids total de la composition de soins bucco-dentaires ; et
un agent de régulation ou de neutralisation du pH ;
dans laquelle la composition de soins bucco-dentaires présente un pH compris entre environ 4 et environ 6.

2. Composition de soins bucco-dentaires selon la revendication 1, dans laquelle l'agent structurant est essentiellement constitué de Carbopol 974 NF.

**3.** Composition de soins bucco-dentaires selon la revendication 1, dans laquelle la composition de soins bucco-dentaires a un pH compris entre 4,8 et 5,8.

**4.** Composition de soins bucco-dentaires selon la revendication 1, dans laquelle la composition de soins bucco-dentaires a un taux de transmission d'au moins 68 % pour la lumière dans la longueur d'onde comprise entre 400 nm et 420 nm, et
dans laquelle la composition de soins bucco-dentaires a un taux de perte de transmission de 1 % ou moins à l'interface de surface, due à la réfraction de la lumière dans la longueur d'onde comprise entre 400 nm et 420 nm.

**5.** Composition de soins bucco-dentaires selon la revendication 1, comprenant en outre :

0,01 % en poids ou moins d'un stabilisant, par rapport au poids total de la composition de soins bucco-dentaires ;
de 0,5 % en poids à 2,0 % en poids d'un agent de régulation de pH, par rapport au poids total de la composition de soins bucco-dentaires ;
de 0,1 % en poids à 2,0 % en poids d'un arôme, par rapport au poids total de la composition de soins bucco-dentaires ; et
de 0,1 % en poids à 5,0 % en poids d'un agent d'hypersensibilité, par rapport au poids total de la composition de soins bucco-dentaires,
dans laquelle l'arôme est soluble dans l'eau et n'est pas à base d'huile.

**6.** Composition de soins bucco-dentaires selon la revendication 5, comprenant :

0,01 % en poids ou moins de stabilisant ;
de 0,5 % en poids à 2,0 % en poids de l'agent de régulation de pH ;
dans laquelle le stabilisant comprend de l'EDTA, l'agent de régulation de pH comprend de la NaOH et l'agent d'hypersensibilité comprend du nitrate de potassium.

**7.** Composition de soins bucco-dentaires selon la revendication 1, constituée essentiellement de :

environ 3 % en poids d'un agent structurant comprenant au moins un élément parmi le Carbopol 974 NF, le Carbopol 980 NF et le Carbopol 971P NF, par rapport au poids total de la composition de soins bucco-dentaires ;
de 30 % en poids à 35 % en poids d'un humectant comprenant de la glycérine, par rapport au poids total de la composition de soins bucco-dentaires ;
de 6 % en poids à 9 % en poids d'un agent de blanchiment comprenant du peroxyde d'hydrogène, par rapport au poids total de la composition de soins bucco-dentaires ; et
au moins 40 % en poids d'un support aqueux, par rapport au poids total de la composition de soins bucco-dentaires ;
dans laquelle tous les ingrédients de la composition de soins bucco-dentaires sont oralement acceptables et solubles dans l'eau.

**8.** Composition de soins bucco-dentaires selon la revendication 7 comprend en outre :

0,01 % en poids ou moins d'un stabilisant comprenant de l'EDTA ;
de 0,5 % en poids à 2,0 % en poids d'un agent de régulation de pH comprenant de la NaOH ;
de 0,1 % en poids à 2,0 % en poids d'un arôme soluble dans l'eau ; et
de 0,1 % en poids à 5,0 % en poids d'un agent d'hypersensibilité comprenant du nitrate de potassium.

**9.** Composition de soins bucco-dentaires selon la revendication 7, dans laquelle l'agent structurant comprend du Carbopol 974 NF.

**10.** Composition de soins bucco-dentaires selon la revendication 7, dans laquelle la composition de soins bucco-dentaires est un gel de blanchiment dentaire conçu pour une utilisation avec une gouttière buccale et une source de lumière.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2883529 A1
- WO 2006071675 A1
- US 6106068 A
- US 5985249 A
- US 6309625 A